# EUROPEAN PATENT APPLICATION

(11) **EP 1 865 317 A1**
(43) Date of publication of application: **12.12.2007**
(21) Application number: 06290951.0
(22) Date of filing: 09.06.2006
(51) Int. Cl.: G01N 33/50, C12Q 1/02, C12N 5/00

(54) **In vitro assay based on cAMP levels modulated by Gi-coupled receptors**

(71) Applicant: Cerep, 75008 Paris (FR)
(72) Inventor: The designation of the inventor has not yet been filed
(74) Representative: Tezier Herman, Béatrice

(57) **Abstract**

The present invention relates to a reliable and effective in vitro assay based on cAMP levels modulated by Gi-coupled receptors expressed in a selected cell for screening and identifying pharmaceutically effective compounds that specifically interact with and modulate the activity of a cellular receptor.

SEQUENCE LISTING

<110>  CEREP

<120>  IN VITRO ASSAY BASED ON CAMP LEVELS MODULATED BY Gi-COUPLED
RECEPTORS

<130>  B0484EP

<160>  21

<170>  PatentIn version 3.3

<210>  1
<211>  29
<212>  DNA
<213>  artificial

<220>
<223>  degenerate primer


<220>
<221>  misc_feature
<222>  (15)..(15)
<223>  r is a or g

<220>
<221>  misc_feature
<222>  (18)..(18)
<223>  h is a or c or t

<220>
<221>  misc_feature
<222>  (21)..(21)
<223>  r is a or g

<220>
<221>  misc_feature
<222>  (24)..(24)
<223>  n is i (inosine)

<220>
<221>  misc_feature
<222>  (27)..(27)
<223>  h is a or c or t

<400>  1
cggcagctcg agaarathaa racnathgg                                          29


<210>  2
<211>  32
<212>  DNA
<213>  artificial

<220>
<223>  degenerate primer


<220>
<221>  misc_feature
<222>  (15)..(15)
<223>  r is a or g

<220>
<221>  misc_feature
<222>  (18)..(18)
<223>  n is i (inosine)

<220>
<221> misc_feature
<222> (21)..(21)
<223> n is i (inosine)

<220>
<221> misc_feature
<222> (24)..(24)
<223> n is i (inosine)

<220>
<221> misc_feature
<222> (26)..(26)
<223> y is c or t

<220>
<221> misc_feature
<222> (27)..(27)
<223> n is i (inosine)

<400> 2
ccgggactcg agacrttnac ngtntynccc ca 32

<210> 3
<211> 258
<212> DNA
<213> Hamster sp.

<400> 3
agaagatcaa aacgataggc agcacctaca tggctgcctc tgggctaaat gccagcacct 60

atgatcaggt gggccgttcc cacatcactg ccctggcaga ctatgccatg aggctcatgg 120

agcagatgaa acatatcaac gaacactctt tcaacaattt ccagatgaag attggactga 180

acatgggtcc agttgtggcc ggtgtcattg gggcccggaa gccacagtat gacatctggg 240

gcgacaccgt caacgtct 258

<210> 4
<211> 270
<212> DNA
<213> Hamster sp.

<400> 4
gagaaaataa aaacgatcgg cagcacctat atggctgcag cagggctcag catcccctca 60

ggacacgaga accaggatct ggagcggcag catgcgcaca ttggtgtctt ggtggagttc 120

agcatggccc tgatgagcaa gctggatggc atcaacaggc actccttcaa ctctttccgc 180

ctccgagttg gcataaacca tgggcctgtg attgctggag tgattggagc acgaaagcct 240

cagtatgaca tctggggcga caccgtcaac 270

<210> 5
<211> 21
<212> DNA
<213> artificial

<220>
<223> sense primer for amplification of fragment 43-805 of mouse
adenylyl cyclase isoform VII

<400> 5

atgccagcca aggggcgcta c                                                21

<210>  6
<211>  20
<212>  DNA
<213>  artificial

<220>
<223>  antisense primer for amplification of fragment 43-805 of mouse
       adenylyl cyclase isoform VII

<400>  6
agatgctgac attctggtgc                                                   20

<210>  7
<211>  23
<212>  DNA
<213>  artificial

<220>
<223>  sense primer for amplification of fragment 628-1618 of mouse
       adenylyl cyclase isoform VII

<400>  7
cacaagcacc agctgcagga cgc                                               23

<210>  8
<211>  21
<212>  DNA
<213>  artificial

<220>
<223>  antisense primer for amplification of fragment 628-1618 of mouse
       adenylyl cyclase isoform VII

<400>  8
agacttggag cagcagggcc t                                                 21

<210>  9
<211>  21
<212>  DNA
<213>  artificial

<220>
<223>  sense primer for amplification of fragment 1685-2816 of mouse
       adenylyl cyclase isoform VII

<400>  9
tgacgagatg ctgtcagcca t                                                 21

<210>  10
<211>  23
<212>  DNA
<213>  artificial

<220>
<223>  antisense primer for amplification of fragment 1685-2816 of mouse
       adenylyl cyclase isoform VII

<400>  10
tgccaatggt cttgatcttc tcc                                               23

<210>  11

<211>  763
<212>  DNA
<213>  Hamster sp.

<400>  11
ggccccaacc aggcaaagct ctatgagaag taccgcctca ccagccagca tgggccactg      60

ttgctcttgc tcctcctggt ggctgtggcc acctgcatcg cactcatcat catcaccttc     120

agccatgagg atccccccag acaccaggct atcctgggca ctgcgttctt catgctgacg     180

ctgtttgtgg ctctctatgt gctggtatat gttgagtgcc tggtgcggcg atggctgtgg     240

gccttggctc tactcacctg ggcctgcctc atgatgctcg gctttgtgct ggtgtgggac     300

tcctggaaga atgatgcctg tgcgtgggag caggtgcctt tcttcctgtt cattgtcttt     360

gtggtgtatg cattactgcc cctcagcacg agggcagcca tcacagtggg gatggtttcc     420

actgtttccc acctcctgat gtttggagct gtgacaggag ccttcaagat gtccgtgtcc     480

ggagcacagc tggcgctgca gctcctggcc aatgccatta tcctcctggg tgggaacttc     540

acgggtgcct tccacaagca ccagctgcag gatgcttccc gggacctctt catctacaca     600

gtcaagtgca tccagatccg ccggaagctg cgtgtggaga agcgccagca ggagaacctg     660

ctgctttcgg tgctcccagc tcacatatcc atgggcatga agctggccat cattgagcgc     720

ctcaaagagg gtggtgacca ccgccacatg cctgacaaca act                       763

<210>  12
<211>  1034
<212>  DNA
<213>  Hamster sp.

<400>  12
ctcttcatct acacagtcaa gtgcatccag atccgccgga agctgcgtgt ggagaagcgc      60

cagcaggaga acctgctgct ttcggtgctc ccagctcaca tatccatggg catgaagctg     120

gccatcattg agcgcctcaa agagggtggt gaccaccgcc acatgcctga caacaacttt     180

cacagcctct atgtcaagcg gcaccagaat gtcagtatct tgtatgcaga cattgtgggc     240

ttcacacggc tggccagtga ctgctcgccc aaggagctgg tggtggtact caacgagctg     300

ttcgggaaat ttgaccagat tgccaaggcc aatgagtgca tgcggatcaa gatcctgggt     360

gactgctact actgtgtgtc aggcctgcct gtgtcgctgc ccacccatgc ccgcaactgc     420

gtgaagatgg gtctggacat ctgtgaggcc attaagcagg ttcgtgaggc cacaggagtg     480

gacatcagca tgcgtgtggg tatccactcc gggaatgttc tgtgtggggt catcgggctc     540

cgcaagtggc agtatgatgt gtggtcccat gatgtgtccc tggccaacag gatggaggca     600

gctggagtcc ctggccgggt gcacatcaca gaggcgacac tgaaccacct ggacaaggca     660

tacgaggtgg aggatgggaa cgggcagcag cgtgacccct atctgaaaga gatgaacatc     720

cgaacctacc tggtgatcga cccccggagc cagcagccgc ctccacccag ccatcacctc     780

cccaagccca agggggatgc aaccctgaag attcgggctt cagtgcgtgt aacgcgctat     840

ctcgagtcct ggggggcagc acggcccttc gcacacctca accaccggga gagtgtgagc     900

agcagcgaga cccccagtcc cactggacag aggccaaaag ccactcctct gcgccgacac     960

cgtgccattg acaggagtac atcccccaag gggcgcttgg aggatgactg tgatgacgag 1020

atgctctcgg ccat 1034

<210> 13
<211> 1132
<212> DNA
<213> Hamster sp.

<400> 13
cctgctgctc caagtccgat gacttccaca cctttggtcc catcttcctg gagaagggct 60

ttgagcgtga gtatcgcctg gtgcctatcc cccgggctcg ctacgacttt gcctgtgcca 120

gccttgtctt catctgcatc ctgcttgtcc accttcttgt gatgcccagg atggccactc 180

tgggcgtgtc ctttggactg gtggcctgcc tgctggggct ggtgctgagt ttctgctttg 240

ctactgagtt ctcaaggtgc tttccagccc gaggtacact ccaggccatc tcggagagtg 300

tggagacaca gcccctgctc aggctatcct tggttgtgct gactgttggt agcctactga 360

ctgttgccat catcaacatg ccactgatgc ttagcccagg cccagagtgg ccaggaggca 420

atgagacaag ctcgctggct gcaaggaata gagttggaat cccgtgtgag ctcctcctgt 480

actacacctg cagctgcatc ctgggcttca tctcgtgctc cgttttcctg cggatgagcc 540

tggaactgaa ggccctgctg ctgacagtgg ccttggtggc ttacctgctg ctcttcaacc 600

tctccccctg ctggcatgtt ccaggcaaca gcactgaaac caacagtaca cacaggacac 660

aaacactcct gcctgctgga caaagcacac ccagccacac ccttgctctg ggggctcagg 720

tgactgcctc ctcccctggc agatgtttag agaaagatct gaagaccatg atcaacttct 780

acctggtcct gttctatgcc accctcatct tgctgtctag acagattgac tactattgcc 840

gcttggattg tctgtggaag aagaagttca agaaagagca tgaggagttt gaaacaatgg 900

agaatgtgaa ccgcctcctc ctggaaaatg ttctgccagc gcatgtggct gcccacttca 960

ttggcgacaa ggcggctgag gactggtacc atcagtctta tgactgtgtc tgtgtcatgt 1020

ttgcctcggt tccggacttc aaagtgttct acactgagtg cgatgtcaac aaagaagggt 1080

tggagtgcct tcgcctgctg aatgagatca ttgctgattt tgacgagctg ct 1132

<210> 14
<211> 19
<212> DNA
<213> artificial

<220>
<223> sequence for miRNA targeted inactivation of adenylyl cyclase
isoform VII

<400> 14
acaactttca gcctctatg 19

<210> 15
<211> 19
<212> DNA
<213> artificial

<220>
<223>  sequence for miRNA targeted inactivation of adenylyl cyclase
      isoform VII

<400>  15
tggtgctgtt tctgctttg                                                    19


<210>  16
<211>  19
<212>  DNA
<213>  artificial

<220>
<223>  sequence for miRNA targeted inactivation of adenylyl cyclase
      isoform VII

<400>  16
tgacaggact tcaagatgt                                                    19


<210>  17
<211>  19
<212>  DNA
<213>  artificial

<220>
<223>  sequence for miRNA targeted inactivation of adenylyl cyclase
      isoform VII

<400>  17
tatgatgtgg tcccatgat                                                    19


<210>  18
<211>  19
<212>  DNA
<213>  artificial

<220>
<223>  sequence for miRNA targeted inactivation of adenylyl cyclase
      isoform VII

<400>  18
ttccaggcca gcactgaaa                                                    19


<210>  19
<211>  19
<212>  DNA
<213>  artificial

<220>
<223>  sequence for miRNA targeted inactivation of adenylyl cyclase
      isoform VII

<400>  19
aaagaaggtg gagtgcctt                                                    19


<210>  20
<211>  19
<212>  DNA
<213>  artificial

<220>
<223>  sequence for miRNA targeted inactivation of adenylyl cyclase
      isoform VII

<400>  20
tcctcctgct acacctgca                                                    19


<210>  21
<211>  19
<212>  DNA
<213>  artificial

<220>
<223>  sequence for miRNA targeted inactivation of adenylyl cyclase
       isoform VII

<400>  21
agcactgacc aacagtaca                                                    19

## Description

### FIELD OF THE INVENTION

The present invention relates to an in vitro assay based on cAMP levels modulated by Gi-coupled receptors.

### DESCRIPTION OF THE PRIOR ART

G-protein-coupled receptors (GPCR) represent one of the largest super families in the human genome. They are involved in a large number of physiological disorders and diseases and therefore represent a significant target for pharmaceuticals and drug discovery. Approximately 60% of all drugs currently in development target GPCRs (Lundstrom et al., Trends Biotechnol., 2005, 23, 103-108).

GPCRs, characterised by their 7 transmembrane spanning domains, can be activated by a wide variety of extracellular stimuli, such as neurotransmitters, hormones, light and odorants. Heterotrimeric G proteins consist of 3 subunits: the α subunit (35kDa) family, the β subunit (35-36kDa) family and the y (6-10kDa) subunit family. Agonist binding leads to active conformational adaptation of the receptor and subsequent activation of G proteins through the association of guanosine-5'-triphosphate (GTP) to the Gα subunit in exchange for guanosine-5'-diphosphate (GDP) (Kristiansen, K., Phamacol Ther, 2004, 103, 21-81). Then, dissociation of the Gα from the Gβγ dimer leads to activation, by Gα or Gβγ dimer, of downstream effectors such as adenylyl cyclases, ion channels and phospholipases.

A diverse array of adapted assays is available, covering exploitation of the three main steps in the GPCR signalling process, including ligand binding and functional assays (for review, Greasley P.J. and Jansen, F.P., Drug Discovery Today: Technologies, 2005, 2, 2, 163-169). These steps include the binding of the ligand to the receptor, the ligand-mediated stabilisation of the receptor in an active conformation resulting in nucleotidic exchange (GTP for GDP in the Gα subunit) and finally, the subsequent coupling to different intracellular secondary messenger pathways.

G proteins coupled to GPCRs include four families Gq, Gs, Gi and G_{12/13} (for review, Wong, S.K.-F., Neurosignals, 2003, 12, 1-12) and coupling to a specific secondary messenger pathway will depend on the G-protein preference of the receptor and of the nature of the agonist used (Akam, E.C. et al., Br J PharmacoL, 2001,132, 4, 950-8; Wong, S. K.-F., Neurosignals, 2003, 12, 1-12) .

Activation of the Gq-coupled receptors leads to the activation of phospholipase C inducing release of calcium stores via the generation of inositol 1,4,5-triphosphate (IP3). Functional assays of this pathway are mainly cell-based and exploit calcium and IP3 signals.

Activation of Gs and Gi-coupled receptors lead to activation (Gs) or inhibition (Gi) of adenylyl cyclase, resulting in increased or decreased intracellular cAMP levels, respectively. Many types of assay formats using fluorescence or luminescence readout can be applied to the measurement of intracellular cAMP (For review, Williams, C. et al., Nat Rev Drug Discov., 2004, 3, 2,125-35). They provide good results for the Gs-coupled receptors.
Nevertheless, it is more challenging to obtain a workable assay for the Gi-coupled receptors using these technologies. Detection of the effects induced by Gi-coupled receptors requires pre-activation by forskolin to stimulate adenylyl cyclase and to produce cAMP and inhibition of cAMP production mediated by binding of an agonist on a Gi-coupled receptor rarely exceeds 60% of the stimulation by forskolin. Furthermore, measuring the reversal by an antagonist of the cAMP decrease mediated by a Gi-receptor agonist as a result of prior forskolin stimulation of the adenylyl cyclase activity yet increases the complexity (Greasley P.J. and Jansen, F.P., Drug Discovery Today: Technologies, 2005, 2, 2, 163-169).

One way to overcome these limitations is to transform normal pharmacological effector of Gi-coupled receptors from adenylyl cyclase to PLCβ pathway by using chimeric (Gqi5) or promiscuous G proteins (G_{16/15}), (Lui, A.M., et al., J. Biomol. Screen, 2003, 8, 39-49). However, the use of non-cognate G proteins has been shown to induce significant modifications of the receptor / G-protein interactions thereby decreasing the relevance of the assay.

Other approaches for assaying cAMP concentration include reporter gene assays (Kent, T.C et al., Journal of Biomolecular Screening, 2005, 10, 5, 437-446). However in this case, measurement of a secondary event, here the activation or inhibition of a reporter gene, favours the generation of false positive and negative hits in a screening.

After the original cloning of the mammalian adenylyl cyclase by Krunpinski et al (Krunpinski et al., Science, 244, 1558-1564), at least nine isoforms of mammalian adenylyl cyclases have been cloned. Several adenylyl cyclase families have been identified based on sequence similarities and regulatory properties, revealing diverse regulation by G proteins, calcium and phosphorylation (Sunahara et al., Ann. Rev. Pharmacol. Toxicol., 1996, 36, 461-480). These intracellular modulators affect the adenylyl cyclase isoenzymes differently. Therefore, the cAMP response upon the activation of a receptor is dependent on the cellular adenylyl cyclase isoenzymes pool present in a given cell.

Mammalian cell lines have a cellular machinery adapted for folding and posttranslational modification of expressed receptor proteins. Therefore, cell lines as chinese hamster ovary (CHO) and human embryonic kidney (HEK) are extensively used as tools for screening and identifying chemical entities that can be developed into drugs. However, specificity of the coupling properties of GPCR expressed in a cell line are often less effective than in their native environment (Kenaskin, T., Pharmaco/. Rev., 1996, 48, 413-463). In addition, the outcome of each step in the signal transduction cascade is dependent on the regulatory properties of the particular isoenzyme pool present in the cells (Varga E.V. et al., European Journal of Pharmacology, 1998,348, R1-R2).

In mammalian cells, different isoforms of adenylyl cyclases have been identified, which are tissue-specifically expressed. The first study to identify the role of adenylyl cyclase isoforms in mammalian cell lines used in the signaling pathways of GPCRs was performed by Varga E.V. et al., in 1998. They used reverse transcriptase polymerase chain reaction (RT-PCR) to amplify and then identify and sequence the adenylyl cyclase cDNA in CHO and B12 cells. They identified adenylyl cyclase isoforms VI and VII in CHO and adenylyl cyclase isoforms III, VI and IX in B12 cells (Varga E.V. et al., European Journal of Pharmacology, 1998,348, R1-R2). Adenylyl cyclase isoforms II, III, VI and IX were also reported In HEK-293 cells (Helleveo, M. et al., Biochem. Biophys. Res. Commun., 1993, 192, 311).

Transfection studies on the sensitivity of adenylyl cyclase isoforms to activation by Gsα or to inhibition by Giα provided precious informations concerning the integration of signalling at the effector level (Sunahara, R.K., et al., Ann. Rev.Pharmacol. Toxicol. 1996, 36, 461-480; Cooper,D.M. et al., Adv. Sec. Mess. Phosph. Res.,1998, 32, 23-51; Simonds, W.F, Trends Pharmacol Sci., 1999, 20, 2, 66-73. 1999). At least 18 different subtypes of Gα subunits have been identified. In particular, the Gi/o subfamily that inhibits adenylyl cyclase and regulates ion channels includes Gᵢ₁, Gᵢ₂, Gᵢ₃, Gₒ₁, Gₒ₂, Gₒ₃, G_{z}, G_{t1.} Gₜ₂ and G_{gust·}

Noteworthy, it was shown that Gαi does not inhibit all the adenylyl cyclase isoforms and that only adenylyl cyclase isoforms I, III, V and VI are sensitive to the Gi family.

Another information concerns the impact of the pre-required forskolin activation on the measurement of the Gi effect in a cAMP based GPCR cellular assay. This molecule is widely used as an in vitro activator of cAMP formation. It has been shown to directly activate almost all the adenylyl cyclase isoforms and to raise cyclic AMP levels in a wide variety of cell lines (Seamon, K.B.et al., J Cyclic Nucleotide Res., 1981, 7, 4, ,201-24). At present, only adenylyl cyclase isoform IX has been identified not to be stimulated by forskolin.

As set forth above, many types of technologies are applied for measuring changes in cAMP concentration providing useful tools for studying Gs-coupled receptors. However, the need for pre-activation by forskolin which directly activates adenylyl cyclase to detect the effect of Gi limits the development of a relevant, working cAMP functional assay for screening and profiling Gi-coupled receptors. The non homogeneous expression pattern of the nine principal identified adenylyl cyclase isoforms accounting for distinctive cell- and tissue-specific patterns of adenylyl cyclase responsiveness, the sensitivity of only certain adenylyl cyclase isoforms to inhibition by Gi proteins as well as multiple G protein coupling subtypes of a significant number of GPCRs (Wong, S.,K.-F, Neurosignals, 2003, 12, 1-12) added to the pre-required forskolin activation to detect the Gi effect, all significantly contribute to the encountered difficulties in the development of a working cAMP functional screening assay dedicated to Gi-coupled receptors.

The difficulties encountered in the development of a relevant, working cAMP functional screening and profiling assay dedicated to selective Gi-coupled receptors represents today a breakthrough in the successful identification of new drug candidates for these receptors. Development of methods allowing overcoming these limitations is of strategic importance to the identification of hits and leads for Gi-coupled receptors.

### SUMMARY OF THE INVENTION

It is an objective of the present invention to solve the aforementioned problems in the art.

Accordingly, in a first aspect, the present invention provides a method for screening, identifying or selecting test compounds using an in vitro assay based on cAMP levels modulated by Gi-coupled receptors expressed in a cell comprising the steps of:
(a) optionally identifying, in a selected cell, the Gαi and adenylyl cyclase isoforms which are involved in the modulation of the intracellular cAMP levels,
(b) favouring the influence of adenylyl cyclase isoform(s) stimulated by forskolin and sensitive to inhibition by Gαi relative to adenylyl cyclase isoform(s) stimulated by forskolin but not sensitive to inhibition by Gαi in said selected cell;
(c) contacting said cell with forskolin and a test compound; and
(d) measuring cAMP levels resulting from contacting said test compound with said cell.

In a second aspect, the invention provides an assay system or a kit for measuring the modulation of cAMP levels by test compounds that specifically interact with and modulate the activity of a cellular receptor, said assay system or kit comprising cells which mainly express adenylyl cyclase isoform(s) stimulated by forskolin and sensitive to inhibition by Gαi.

In another aspect, the invention provides a recombinant cell engineered from a selected cell expressing both adenylyl cyclase isoform(s) stimulated by forskolin and sensitive to Gαi and adenylyl cyclase isoform(s) stimulated by forskolin and not sensitive to Gαi, wherein the influence of adenylyl cyclase isoform(s) stimulated by forskolin and sensitive to Gαi is favoured relative to the influence of adenylyl cyclase isoform(s) stimulated by forskolin and not sensitive to Gαi.

The invention further provides the use of a recombinant cell as defined above as a tool for screening, identifying or selecting potential ligands of Gi-coupled receptors.

### DESCRIPTION OF THE INVENTION

Before further description of the invention, certain terms employed in the specification, examples and claims are defined below.

### Definitions

A "DNA construct" as used herein means a polynucleotide which contains an open reading frame which encodes one or more protein(s) and includes any promoter, transcription initiation, transcription termination, or other nucleotide sequences operatively linked to said polynucleotide which might facilitate expression of the encoded protein(s).

The term "heterologous DNA", "heterologous nucleic acid" or "foreign DNA" relates to DNA that does not occur naturally as part of the genome in which it is present. Generally, although not necessarily, such DNA encodes proteins that are not normally produced by the cell in which it is expressed. Heterologous DNA can be from the same species, although in preferred embodiments, it is from a different species. Any DNA that one of skill in the art would recognize or consider as heterologous or foreign to the cell in which it is expressed is herein encompassed by the term heterologous DNA.

The term "cells" as used herein refers to all normal (natural) and transformed cells derived from, for example eukaryotes such as mammalian cells including hybridoma cells, insect cells, plant cells, yeast cells. Examples of mammalian species include primates (e.g., humans, monkeys, chimpanzees, baboons), rodents (e.g., rats, mice, guinea pigs, hamsters) and ruminants (e.g., cows, horses). Mammalian cells may be derived from any recognized source with respect to species (e. g. human, rodent), tissue source (brain, liver, lung, heart, kidney, skin, muscle) and cell type (e. g. epithelial, endothelial). In addition, transfected cells expressing recombinant genes may also be used in the present invention.

Cells to be used in the practice of the methods of the present invention may be primary cells, secondary cells or immortalized cells (i.e., established cell lines). They may be prepared by techniques well known in the art or purchased from immunological and microbiological commercial sources (for example, from the American Type Culture Collection, Manassas, VA). Alternatively or additionally, cells may be genetically engineered to contain, for example, a gene of interest such as a gene expressing an adenylyl cyclase isoform, a Gαi protein or a receptor.

Non-limiting examples of useful mammalian cell lines include animal and human cell lines such as Chinese hamster ovary (CHO) cells, Chinese hamster lung (CHL) cells, baby hamster kidney (BHK) cells, COS cells, HeLa cells, THP cell lines, TAg Jurkat cells, hybridoma cells, carcinoma cell lines, hepatocytes and the like.

"Stably transfected" as used herein means the introduction and maintenance of exogenous DNA into a cell, or cell line, by integration into the genome of the host cell, e.g. into the host chromosomes or by episomal transfection for at least 2 weeks and preferably more than 4 months.

"Transiently transfected" as used herein means that the transfected exogenous DNA is not integrated in the genome of the host cell.
DNA constructs that express siRNAs can be transiently transfected into mammalian cells using standard techniques. However, cells vary greatly with respect to their capacity to be transfected. Hence, different transfection reagents and cell culture conditions need to be tested for each cell type.

The term "modulation" as in "modulation of the intracellular cAMP levels" is intended to encompass modulation in its various grammatical forms (e.g., "modulated", "modulation", "modulating", etc.) and encompass induction, stimulation, potentiation, inhibition of one or more signal transduction pathways downstream of a receptor.

The term "inactivation" or "inactivate" relates to the inhibition, either total or partial, of the expression of a gene (also termed downregulation or knockdown) or of the activity of a protein. For example, this term means that production of a functional gene product is prevented or inhibited. Inactivation may be achieved, for example, either by deletion of the gene (also termed knockout) or mutation of the promoter controlling a gene so that expression does not occur, or mutation of the coding sequence so that the gene product is inactive, or by "RNAi" technique.

The term "RNAi" or "RNA interference" technique refers to the post-transcriptional process of gene silencing mediated by double stranded RNA (dsRNA) that is homologous in sequence to a RNA to be silenced and is observed in a wide variety of eukaryotic cells such as animal and plant cells. Depending on the cell type, dsRNA may be processed into 21-23 nucleotides (nts) molecules, called small interfering RNAs (siRNAs), which guide the sequence-specific degradation of the target RNA (Sharp, 2001). Furthermore, transfection of siRNA specific to a target gene of which expression is to be inactivated, or of a vector allowing the expression of such a siRNA in a cell, in particular in mammalian cells, has been proven to be a potent tool in mammalian genetic studies as such specific siRNA induce the downregulation of the expression of the target gene (Elbashir et al., Nature. 2001 May 24;411(6836):494-8; "Duplexes of 21-nucleotide RNAs mediate RNA interference in cultured mammalian cells."). Furthermore, RNAi can be used for therapeutic applications, diagnostic and target validation (EP Patent n°1144623 ; EP Patent1214945; EP Patent 1352061; US Patent 5898031; US Patent 6107094).
The term "siRNA" as used herein means a double stranded short interfering RNA molecule, designed in vitro, not larger than about 23 nucleotides in length, and preferably of 19-23 nucleotides in length. The scientific literature describes siRNA as mediating the sequence specific degradation of a target mRNA.

The term "miRNA" as used herein means endogenous 15-35 nucleotides, preferably 19-30 nucleotides, most preferably 21-24 nucleotides small RNAs processed from stem-loop RNA precursors (pre-miRNA). miRNA can downregulate gene expression by two posttranscriptional mechanisms: mRNA cleavage or translational repression.

The term "Stem-loop" as used herein means a single stranded polynucleotide including two complementary base sequences separated by a sequence with no complementary base sequence thereof and that allow formation of a duplex structure in the corresponding polyribonucleotide, with a single-stranded loop sequence linking two annealed base sequences. The complementary base sequences forming the stem portion of the stem-loop consist of at least two and more preferably at least three base pairs in length. In particular embodiments, where the stem base pair(s) correspond to the complementing sequence of the first and third sequences, one or more of the second sequence complementing stem base pairs can double as a first and third sequence complementing base pair. Under these special circumstances, the stem portion of the second sequence would be considered to contain only one complementing base pair, or no complementing base pairs.

Preferred stem-loop sequences are based on stem-loop regions known to those persons skilled in the art to be present in RNA molecules such as tRNA, snRNA, rRNA, mtRNA, or structural RNA sequences. One skilled in the art can readily identify stem-loop RNA structures using predictive computer modelling programs such as Mfold (M. Zuker, D. H. Mathews & D. H. Turner Algorithms and Thermodynamics for RNA Secondary Structure Prediction: A Practical Guide In RNA Biochemistry and Biotechnology, 11-43, J. Barciszewski & B. F. C. Clark, eds, NATO ASI Series, Kluwer Academic Publishers, (1999)), RNAstructure (Mathews, D. H.; Sabina, J.; Zuker, M.; and Turner, D. H. ,"expanded sequence dependence of thermodynamic parameters improves prediction of RNA secondary structures", Journal of Molecular Biology, 1999,288, 911-940), RNAfold in the Vienna RNA Package (Ivo Hofacker, Institut fur theoretische Chemie,Wahringerstr. 17, A-1090 Wien, Austria), Tinoco plot ( Tinoco, I. Jr. , Uhlenbeck,O. C. & Levine, M. D. (1971) Nature 230, 363-367), ConStruct, which seeks conserved secondary structures (Luck, R. , Steger, G. & Riesner, D. (1996), Thermodynamic prediction of conserved secondary structure: Application to RRE-element of HIV, tRNA-like element of CMV, and mRNA of prion protein.J. Mol. Biol. 258, 813-826; and Luck, R. , Graf, S. & Steger, G. (1999), Construct : A tool for thermodynamic controlled prediction of conserved secondary structure. Nucleic Acids Res. 21, 4208- 4217), FOLDALIGN, (J. Gorodkin, L. J. Heyer and G. D. Stormo. Nucleic Acids Research, Vol. 25, no. 18 pp 3724-3732,1997a ; and J. Gorodkin, L. J. Heyer, and G. D. Stormo. ISMB5; 120-123,1997b), and RNAdraw (Ole Matzura and Anders Wennborg Computer Applications in the Biosciences (CABIOS), Vol. 12 no. 3 1996, 247-249).

The term "compound" as used herein (e.g., as in "test compound") is meant to include exogenously added test compounds. The test compound may be contacted with a cell. Exemplary compounds which can be screened for activity include, but are not limited to, peptides, nucleic acids, carbohydrates, small organic molecules and natural product extract libraries. Both agonizing and antagonizing compounds are intended to be screened, identified or selected in the present invention.

The term "active compounds" as used herein refers to pharmaceutically effective compounds that specifically interact with, and modulate, the activity of a cellular receptor. This term is equivalent to ligand.

As used herein, the term "effective amount" refers to an amount of a modulator compound, or pharmaceutical composition comprising at least one such compound, that is sufficient to achieve an intended purpose. For example, the intended purpose may be to inhibit or enhance the cAMP accumulation or adenylyl cyclase activity in the whole organism of a subject or in (s) particular tissue(s).

### BRIEF DESCRIPTION OF THE DRAWINGS

Figure 1 shows Western Blots characterising Gαi protein in CHO cell.

Figure 2 shows an example of a targeted sequence of SEQ ID N° 10 (ACVII_miR_173) and the miRNA construction to be inserted into the plasmid.

Figure 3 depicts blots that show the suppressive effect of different miRNAs on the expression of adenylyl cyclase VII in CHO-K1 cells.

Figure 4 shows the dose-response curves characterising the measurement of cAMP induced by acetylcholine and quimpirole.

### DETAILED DESCRIPTION OF THE INVENTION

The present invention provides a reliable and effective in vitro cAMP based assay for Gi-coupled receptors expressed in a selected cell for screening and identifying pharmaceutically effective compounds that specifically interact with and modulate the activity of a cellular receptor.

As set forth above, observation of the modulation of cAMP levels in a cell by Gi-coupled receptors requires the prior treatment of the cell with forskolin, an activator of adenylyl cyclase isoenzymes. Taking into account that the cAMP response upon activation is dependent on the cellular isoenzyme pool present in a particular cell or cell line, non specificity of the forskolin mediated adenylyl cyclase isoforms stimulation, required to detect the Gαi effect, may significantly contribute to the lack of efficient inhibition of cAMP production mediated by agonists of Gi-coupled receptors. As a consequence, the resulting agonist mediated inhibition rarely exceeds 60 % of the stimulation by forskolin. This renders difficult to then measure the reversal by an antagonist of the cAMP decrease mediated by a receptor agonist.

The applicant has shown that one way to efficiently improve the agonist inhibition of the cAMP production mediated by forskolin, and therefore to favour easy quantification of an antagonist activity through the reversal of an agonist effect in a particular cell expressing Gi-coupled receptors, is to favour the influence of adenylyl cyclase stimulated by forskolin and sensitive to Gαi protein.

Accordingly, resulting detection of the Gi effect is mainly associated to the specific inhibition, induced by the interaction with an agonist, of the forskolin pre-stimulated adenylyl cyclase isoform activity sensitive to the Gαi family.

Accordingly, in a first aspect, the invention provides a method for screening, identifying or selecting test compounds using an in vitro assay based on cAMP levels modulated by Gi-coupled receptors expressed in a cell comprising the steps of:
(a) optionally identifying, in a selected cell, the Gαi and adenylyl cyclase isoforms which are involved in the modulation of the intracellular cAMP levels,
(b) favouring the influence of adenylyl cyclase isoform(s) stimulated by forskolin and sensitive to inhibition by Gαi relative to adenylyl cyclase isoform(s) stimulated by forskolin but not sensitive to inhibition by Gαi in said selected cell;
(c) contacting said cell with forskolin and a test compound; and
(d) measuring cAMP levels resulting from contacting said test compound with said cell.

Selection of the most appropriate cells comprises both identification of Gαi protein and adenylyl cyclase isoforms which are involved in the modulation of intracellular cAMP levels. Indeed, it has been shown that the expression pattern of the different adenylyl cyclase isoforms is cell- or tissue-specific. Accordingly, for the cells for which no data is available in the litera ture on the expression of adenylyl cyclase isoforms, a first step of identification of the adenylyl cyclase isoforms expressed in the specific cell type selected for use in the invention must be implemented.

To date, among the cloned mammalian adenylyl cyclase, all except isoform IX have been identified as stimulated by forskolin. In addition, only adenylyl cyclase I, III, V and VI are sensitive to the Gαi family.

The choice of an appropriate cell line is influenced by the presence or absence of particular adenylyl cyclase. The selected cell should express at least one adenylyl cyclase isoform shown to be activated by forskolin and sensitive to inhibition by Gαi proteins. In another embodiment, the cell used in the method of the present invention contains only adenylyl cyclase isoforms activated by forskolin and sensitive to inhibition by Gαi proteins, and no adenylyl cyclase isoform activated by forskolin and insensitive to inhibition by Gαi proteins.

An embodiment of the invention consists in selecting cells from the group consisting of natural cells and established cell lines expressing endogenous Gαi protein and adenylyl cyclase isoforms.

In another embodiment of the invention the established cell lines express cloned Gαi protein and/or adenylyl cyclase isoforms.
In a particular embodiment the host cell is a eukaryotic cell selected among a mammalian cell, a yeast cell or an established cell line selected among CHO, HEK or the insect SF9 lines. In a preferred embodiment the host cell is the CHO-K1 or CHO-S line.

In a preferred embodiment, the cells express a number of adenylyl cyclase isoform(s) stimulated by forskolin but not sensitive to inhibition by Gαi protein comprised between 1 and 4, more preferably 4, more preferably 3, more preferably 2 and most preferably 1.
In case adenylyl cyclase isoform(s) stimulated by forskolin but not sensitive to inhibition by Gαi are expressed in the selected cell, they must be inactivated in order to overcome the problem encountered in the methods described in the state of the art, i.e. the poor induction of inhibition of cAMP production mediated by Gi-coupled receptors as a consequence of the activation by forskolin of a number of adenylyl cyclases, including those adenylyl cyclases which are stimulated by forskolin but insensitive to inhibition by Gαi proteins.

Alternatively, the selected cells may correspond to a cell type in which no Gαi protein and/or no adenylyl cyclase isoform is naturally expressed. In this case, exogenous expression of given isoform(s) of the aforementioned isoforms can be obtained by, for example, transient or stable transfection of vectors comprising polynucleotides coding for the specific protein of interest to express.

In a particular embodiment, favouring the influence of adenylyl cyclase isoform(s) stimulated by forskolin and sensitive to inhibition by Gαi relative to adenylyl cyclase isoform(s) sensitive to forskolin but not sensitive to inhibition by Gαi is achieved by specifically inactivating the adenylyl cyclase isoform(s) stimulated by forskolin but not sensitive to inhibition by Gαi protein. Inactivation of such Gαi-insensitive adenylyl isoform(s) can be achieved, for example, by inhibiting specifically their catalytic activity or preferably by downregulating their expression.

While modifying gene expression in a cell in the method of the present invention, one must take care to carry out such modification without affecting cell viability.

The nucleic acid molecules can be inserted into a construct which can, optionally, replicate and/or integrate into the mammalian cell, by known methods to those skilled in this art. In practicing the present invention, many conventional techniques in molecular biology, microbiology, and recombinant DNA can be used. Such techniques are well known and are explained in details in, for example, Sambrook et al., 1989, Molecular Cloning: A Laboratory Manual, Second Edition, Cold Spring Harbor Laboratory Press, Cold Spring Harbor, New York; DNA Cloning : A Practical Approach, Volumes I and II, 1985 (D. N. Glover ed.) ; Oligonucleotide Synthesis, 1984, (M. L. Gait ed.) ; Nucleic Acid Hybridization, 1985, (Hames and Higgins); Transcription and Translation, 1984 (Hames and Higgins eds.) ; Animal Cell Culture, 1986 (R. I. Freshney ed.) ; Immobilized Cells and Enzymes, 1986 (IRL Press); Perbal, 1984, A Practical Guide to Molecular Cloning; the series, Methods in Enzymology (Academic Press, Inc.) ; Gene Transfer Vectors for Mammalian Cells, 1987 (J. H. Miller and M. P. Calos eds., Cold Spring Harbor Laboratory); and Methods in Enzymology Vol. 154 and Vol. 155 (Wu and Grossman, and Wu, eds., respectively).

Step (b) can be achieved in various ways. In an exemplary embodiment, favouring the influence of adenylyl cyclase isoform(s) stimulated by forskolin and sensitive to inhibition by Gαi relative to adenylyl cyclase isoform(s) sensitive to forskolin but not sensitive to inhibition by Gαi is achieved by increasing the expression of adenylyl cyclase isoform(s) gene(s) stimulated by forskolin and sensitive to inhibition by Gαi protein.

In a further embodiment of the invention, any type of genes from the natural cells or established cell lines identified as interfering with Gi-coupled receptor specific pathways can be upregulated or downregulated to favour these specific pathways. In addition, any exogenous gene not present in the selected cell line expressing Gi-coupled receptor(s) of interest and susceptible to enhance the adenylyl cyclase isoforms response to Gi in response to a test compound can be expressed or co-expressed with other endogenous selected genes or any type of oligonucleotides including siRNA or miRNA in order to improve the assay.

It is well known that certain agonists can demonstrate high affinity for G-coupling receptor states due to the promotion of G protein coupling. In many natural receptors systems, the amount of G protein available for formation of such complexes is not limiting and the complete receptor population can be converted into a high affinity ternary complex by an efficacious agonist (Kenanin, T., TiPS, 1997, 18, 456-464). However, in some systems including, for example mammalian cell lines, the availability of the required G proteins as Gi proteins subtypes is limiting. On the other hand, in the context of the invention, the pre-required stimulation of the endogeneous identified adenylyl cyclase isoforms by forskolin may limits the extent of inhibition by Gαi (Dessauer, C., W., The Journal of Biological Chemistry, 2002, 277, 32, 28823-28829). In other hands, according to the invention, one can develop an established stable host cell line in which it is possible to co-express in the same plasmid, specific miRNAs and a specific Gi subtype protein in order to re-enforce the Gαi inhibition of the forskolin and/or Gαs stimulated adenylyl cyclases. To illustrate this, CHO-K1 and CHO-S stably expressing adenylyl cyclase VII specific miRNAs can be used to develop relevant and robust cAMP based cellular assays expressing GPCR coupling the specific Gi protein by co-expressing, preferably in the same plasmid, the specific adenylyl cyclase isoform VII miRNA and a specific Gαi protein specific subtype.

In one embodiment of the present invention the selected cells express the Gi protein of interest endogenously. In yet other embodiments, the cells are engineered to express a heterologous Gi protein. In both cases, it may be desirable to inactivate one or more endogenous genes of the host cells.

According to the method of the present invention Gαi protein are selected among the G_{i/0} subfamily that inhibits adenylyl cyclase and include Gᵢ₁, Gi₂, Gᵢ₃, G₀₁, G₀₂, G₀₃, Gz, Gₜ₁, Gₜ₂ and G_{gust.}

A large number of well-known vectors can be used in the invention for expression in a variety of eukaryotic hosts. For example, the Gαi protein or Gi-coupled receptors may be expressed by transfecting well-known mammalian expression vectors.

One skilled in the art has at his disposal a number of techniques for identifying the expression of a given protein. For example, the identification of adenylyl cyclase isoforms expressed in the selected cell can be performed by PCR, preferably RT-PCR amplification using degenerate primers. Alternatively, one can use western-blot analysis to identify which adenylyl cyclase isoform(s) are expressed in the selected cell by using antibodies specific of each isoform.

The different types of adenylyl cyclase identified to date share a common structure. Each has two domains, consisting of six putative membrane-spanning regions separated by a large cytoplasmic loop, as well as a large cytoplasmic loop at the carboxyl terminus. The different isoforms of adenylyl cyclase are reviewed in Taussig R, Gilman AG. (1995) Mammalian membrane-bound adenylyl cyclases. J Biol Chem 270 and Sunahara RK, Dessauer CW, Gilman AG. (1996) Complexity and diversity of mammalian adenylyl cyclases. Annu Rev Pharmacol Toxicol 36, 461-80.

Various methods are known in the art for identification of nucleic acids having sequence similarity, based on the ability of single stranded nucleic acids, e.g. DNA or RNA oligo- or polynucleotides, to form a double stranded complex by complementarity. A sample may be screened for the presence of specific target nucleic acids capable of forming double stranded complexes with, for example, an adenylyl cyclase probe, in particular under low stringency conditions. "Sample" as used herein includes but is not limited to genomic libraries, cDNA libraries, nucleic acid molecule extracts from tissue, or nucleic acid molecule extracts from cell culture.

After complex formation, the target nucleic acid molecule is isolated by methods known in the art. Methods include the polymerase chain reaction (PCR) or reverse transcriptase PCR (RT-PCR), where two probes specific of a target nucleic acids sequence are used to amplify a region of the sample DNA or cDNA. PCR may also be performed with a single adenylyl cyclase probe, and a second oligonucleotide complementary to a known genomic repeat sequence.

Generally the adenylyl cyclase probe will be labelled with a detectable marker, e.g.³²P, ³⁵S, biotin, FITC, etc. when used in a northern or southern blot.

Nucleic acids used in the invention may be isolated directly from well-known cells. Alternatively, the polymerase chain reaction (PCR) method can be used to produce the nucleic acids of the invention, using either chemically synthesized strands or genomic material as templates. Primers used for PCR can be synthesized using the sequence information provided herein and can further be designed to introduce appropriate new restriction sites, if desirable, to facilitate incorporation into a given vector for recombinant expression.

Multiple sequences from a target mRNA can be selected in order to identify a target sequence which is specific for a particular mRNA. Sequence alignments with other mRNA sequences that have high homology to the target mRNA are used. If the goal is to identify in a selected cell, Gαi or adenylyl cyclase isoforms, gene homology technique can be used. In the particular case of the present invention, sequences of Gi proteins or adenylyl cyclase isoforms are available from Genbank. For example the mouse adenylyl cyclase VI gene (NM_007405) can be used to identify the homologue hamster gene in CHO cells.

The invention comprises the use of natural cells or established cell lines in which the expression of adenylyl cyclase isoform(s) stimulated by forskolin but not sensitive to inhibition by Gαi can be down regulated by any type of approaches. Particularly preferred cells are those in which RNAi technique can be implemented in order to downregulate the expression of given gene. This technique includes the design, the selection and then the use of adapted RNAi as microRNA (miRNA).

Inactivation by RNAi may be obtained either by transiently transfecting adapted RNAi such as siRNA or miRNA specific for a given target gene to be inactivated or by transfecting, either transiently or stably, an expression vector coding such siRNA or miRNA.

In a particular embodiment, expression of a miRNA is desired. The expression of miRNA may be obtained with DNA-based vectors including engineered pre-miRNA primary transcripts. Designed polynucleotide sequences can be inserted for expression in an eukaryotic cell vectors such as, for example, pSilencer 4.1-CMV (Ambion), pcDNA3.1, pcDNA3.1/zeo or pcDNA3.1/hyg, pRc/RSV, pCMV5 or pRc/CMV2, pRNA-CMV3.1 (GenScript Corporation), or U6 promoter derived vectors such pRNA-U6.1 (GenScript Corporation) or pSilencer 2.0-U6 (Ambion), or Hi promoter derived vectors such pRNA-H1.1 (GenScript Corporation) and pSilencer 3.0- H1 (Ambion).

In a particular embodiment of the invention, the designed polynycleotides for expressing miRNA includes a Drosha cleavage site to the hairpin construct, which has been shown to greatly increase knockdown efficiency (Boden *et al* 2004, Silva *et al* 2005).

Advantageously, the hairpin may consist of 19 to 22-nt of dsRNA and a 19-nt loop from human miR30. Furthermore, in another embodiment of the invention, the miR30 loop and/or 125-nt of miR30 flanking sequence on either side of the hairpin results in greater than 10-fold increase in Drosha and Dicer processing of the expressed hairpins when compared with conventional shRNA designs without miRNA adaptations (Silva *et al* 2005). This technique will be further detailed in the examples section.

In certain embodiments the method described herein is used for identifying active compounds for "orphan receptors" for which no ligand is known.

In a particular embodiment of the invention, the method enables rapid screening or profiling of large numbers of test compounds to ascertain their agonist or antagonist activity with respect to Gi-coupled receptors expressed in a cell.

The test compound can interact with a receptor protein which can be any receptor which interacts with said test compound to modulate a signal in the cell. For example, the target receptor may be a cell surface receptor. It must be understood that the method of the invention is a screening method which allow the rapid a reliable identification of compounds potentially having the ability to modulate Gi-coupled receptors.

Preferred G-protein-coupled receptors, include [alpha]1A-adrenergic receptor, [alpha]1B-adrenergic receptor, [alpha]2-adrenergic receptor, [alpha]2B-adrenergic receptor, [beta]1 -adrenergic receptor, [beta]2-adrenergic receptor, [beta]3-adrenergic receptor, m1 acetylcholine receptor (AChR), m2 AChR, m3 AChR, m4 AChR, m5 AChR, Apalin, D1 dopamine receptor, D2 dopamine receptor, D3 dopamine receptor, D4 dopamine receptor, D5 dopamine receptor, A1 adenosine receptor, A2b adenosine receptor, 5-HT1a receptor, 5-HT1b receptor, 5HT1-like receptor, 5-HT1d receptor, 5HT1 d-like receptor, 5HT1d beta receptor, substance K (neurokinin A) receptor, fMLP receptor, fMLP-like receptor, angiotensin II type 1 receptor, endothelin ETA receptor, endothelin ETB receptor, thrombin receptor, growth hormone-releasing hormone (GHRH) receptor, vasoactive intestinal peptide receptor, oxytocin receptor, somatostatin SSTR1 and SSTR2, SSTR3, cannabinoid receptor, follicle stimulating hormone (FSH) receptor, leutropin (LH/HCG) receptor, thyroid stimulating hormone (TSH) receptor, thromboxane A2 receptor, platelet-activating factor (PAF) receptor, C5a anaphylatoxin receptor, Interleukin 8 (IL-8) IL-8RA, IL-8RB, Delta Opioid receptor, Kappa Opioid receptor, mip-1/RANTES receptor, -Rhodopsin, Red opsin, Green opsin, Blue opsin, metabotropic glutamate mGluR1-6, histamine H2 receptor, ATP receptor, neuropeptide Y receptor, amyloid protein precursor receptor, insulin-like growth factor II receptor, bradykinin receptor, gonadotropin-releasing hormone receptor, cholecystokinin receptor, melanocyte stimulating hormone receptor receptor, antidiuretic hormone receptor, glucagon receptor, and adrenocorticotropic hormone II receptor.

In a particular embodiment, the invention provides a method for screening, identifying or selecting test compounds potentially having a Gi-coupled receptor modulating activity in a selected cell by implementing the above described method steps. All Gi-coupled receptors must physically couple to Gi proteins in order to transduce extracellular stimuli into intracellular signals, i.e. the modulation of cAMP levels that lead to functional responses. Accordingly, as to further determine which compounds identified in the method of the invention are ligands of Gi-coupled receptors, further characterisation may be necessary for eliminating those compounds which act on the level of cAMP in the cell by secondary interfering pathways. This characterisation can be achieved, for example, by competitive assays on know Gi-coupled receptors in order to determine which receptor the identified test compounds bind.

According to the present invention, screening or profiling can be performed in a high throughput mode.

In a particular embodiment of the method of the invention, the potential effects of the test compound on Gi-coupled receptors is carried out by comparing cAMP levels in cells treated by forskolin and either treated or untreated by the test compound.

Measurement of intracellular cAMP may be accomplished by well-known in the art detection assays. Among them one can use, for example, the most common automatable technologies used for HTS as SPA (Amersham Biosciences), Flashplates and AlphaScreen (Perkin Elmer life and Analytical Sciences), fluorescence polarisation (Perkin Elmer lifesciences, Amersham Biosciences), DiscovereX/HitHunter, β-Gal EFC (DiscoverX/GE healthcare), Time resolved-FRET (Cisbio International, Perkin Elmer Lifesciences), Electrochemiluminescence (Meso Scale Discovery), CatchPoint (Molecular Devices) ACTOne cAMP assay (BD Biosciences).

Said assays may be, for example, homogeneous or heterogeneous assays including sandwich assays.

In another embodiment of the present invention, measurement of intracellular cAMP level modulation induced by test compounds can be performed by measurement of the changes in intracellular cAMP levels through technologies using radioactivity, colorimetry, fluorescence or luminescence.

In a particular embodiment of the invention, selected cells are disposed in a plurality of wells of a multi-well assay plate. The cells may be, for example, incubated in some wells of the assay plate with a test compound under conditions and for a time sufficient to allow equilibration. The test is then read, for example, with a labelling approach that combines the time-resolved fluorescence (TRF) technique and the fluorescence resonance energy transfer (FRET) technique. FRET refers to a non-radiative energy transfer of excitation energy between suitable donor and acceptor fluorophores.
The amount of fluorescence read is proportional to the adenylyl cyclase activity induced by active compounds, in effective amount, that specifically interact with and modulate the activity of a cellular receptor.

Cells to be used in the inventive assays may be cultured according to standard cell culture techniques. For example, cells are often grown in a suitable vessel in a sterile environment at 37°C in an incubator containing a humidified 95% air - 5% CO2 atmosphere. Vessels may contain stirred or stationary cultures. Various cell culture media may be used including media containing undefined biological fluids such as fetal calf serum, as well as media which are fully defined, such as 293 SFM serum free medium (Invitrogen Corp., Carlsbad, CA). Cell culture techniques are well known in the art and established protocols are available for the culture of diverse cell types (see, for example, R.I. Freshney, "Culture of Animal Cells: A Manual of Basic Technique", 2nd Edition, 1987, Alan R. Liss, Inc.)

In a preferred embodiment, the invention comprises implementing CHO-S or CHO-K1 cells in which adenylyl cyclase VII expression is knocked-down in the method described above for screening, selecting or identifying test compounds.

A further aspect of the invention relates to an assay system or a kit for measuring the modulation of cAMP levels by test compounds that specifically interact with and modulate the activity of a cellular receptor, said assay system or kit comprising cells which mainly express adenylyl cyclase isoform(s) stimulated by forskolin and sensitive to inhibition by Gαi.

The term "mainly express" as used herein denotes the more prevalent influence of Adenylyl cyclase isoform(s) stimulated by forskolin and sensitive to inhibition by Gαi relative to Adenylyl cyclase isoform(s) stimulated by forskolin but not sensitive to inhibition by Gαi, in selected cells, as set forth above.

In a particular embodiment, the assay system or kit of the invention comprises cells in which the expression of adenylyl cyclase isoform(s) stimulated by forskolin but not sensitive to inhibition by Gαi has been inactivated by specific targeting by miRNA.

The assay system or kit of the present invention is implemented by contacting the cells thereof with forskolin and a test compound and then measuring the levels of cAMP resulting from said contacting.

In a particular embodiment, the invention provides an assay system or kit wherein said cell stably co-express i) nucleic acid sequences which code for specific Gαi protein or specific adenylyl cyclase isoform(s) stimulated by forskolin and sensitive to inhibition by Gα i protein (ii) and specific miRNA that target adenylyl cyclase transcripts coding for adenylyl cyclase isoform(s) stimulated by forskolin and not sensitive to inhibition by Gα i protein.

In another particular embodiment, the assay system or kit of the invention, comprises the recombinant cells described hereinabove.

In another aspect, the invention provides a recombinant cell engineered from a selected cell expressing both adenylyl cyclase isoform(s) stimulated by forskolin and sensitive to Gαi and adenylyl cyclase isoform(s) stimulated by forskolin and not sensitive to Gαi, wherein the influence of adenylyl cyclase isoform(s) stimulated by forskolin and sensitive to Gαi is favoured relative to the influence of adenylyl cyclase isoform(s) stimulated by forskolin and not sensitive to Gαi.

According to a particular embodiment, expression of adenylyl cyclase isoform(s) stimulated by forskolin and sensitive to Gαi is upregulated in the recombinant cell of the invention.

In another particular embodiment, adenylyl cyclase isoform(s) stimulated by forskolin and not sensitive to Gαi are inactivated in the recombinant cell of the invention, for example by deletion of the gene(s) coding said isoform(s) or by downregulation by RNAi.

Additionally, different ways to achieve a favoured influence of adenylyl cyclase isoform(s) stimulated by forskolin and sensitive to Gαi relative to the influence of adenylyl cyclase isoform(s) stimulated by forskolin and not sensitive to Gαi have been described hereinabove.

In a particular embodiment of the recombinant cell of the invention, said recombinant cell is transfected, preferably stably, with at least one recombinant expression vector coding for one or more miRNA specific for Adenylyl Cyclase isoform(s) expressed in the cell from which said cell is engineered. In another embodiment, said recombinant expression vector coding for said at least one or more miRNA is co-transfected with a recombinant expression vector comprising one or more nucleic acid sequences coding for specific Gαi protein or specific adenylyl cyclase isoform(s) stimulated by forskolin and sensitive to inhibition by Gαi protein.

The recombinant expression vectors transfected in the recombinant cell of the invention can be an appropriate eukaryotic expression vector, including an engineered pre-miRNA structure. Advantageously, the recombinant expression vectors may further comprise a Drosha cleavage site in the hairpin construct. The hairpin stem preferably consists of 21-22-nt of dsRNA (mature miRNA) derived from the target gene and the hairpin loop may comprise, in a preferred embodiment, 19-nt.

In a particular embodiment of the present invention, a recombinant cell line is engineered which co-express a heterologous Gαi protein and specific miRNA that target adenylyl cyclase transcripts coding for adenylyl cyclase isoform(s) stimulated by forskolin and not sensitive to inhibition by Gαi protein.

In a particular embodiment, the recombinant cell of the invention is a cell stably transformed with a recombinant DNA vector comprising (i) engineered pre-miRNA primary transcripts, ii) nucleic acid sequences which code for a specific Gαi protein or a specific adenylyl cyclase isoform(s) stimulated by forskolin and sensitive to inhibition by Gα i protein, stably co-expressed in said recombinant cell.

In a particular embodiment, the recombinant cell of the invention is a cell engineered to co-express a specific adenylyl cyclase isoform(s) stimulated by forskolin and sensitive to inhibition by Gα i protein and specific miRNA that target adenylyl cyclase transcripts coding for adenylyl cyclase isoform(s) stimulated by forskolin and not sensitive to inhibition by Gα i protein.

In a particular embodiment the recombinant cell is a mammalian cell, for example of the CHO cell line.

The recombinant cell according to the invention can be used as a tool in an in vitro assay based on cAMP levels modulated by Gi-coupled receptors. Accordingly, the invention further provides the use of a recombinant cell as defined above as a tool for screening, identifying or selecting potential ligands of Gi-coupled receptors.

The use of CHO-K1 or CHO-S cells is particularly preferred. These cells are available from the European Collection of Cell Cultures (ECACC), Porton Down, UK Accession Number 85051005. HEK-293 cells are available under Accession Number 85120602 (ECACC) and sf9 cells under Accession Number 89070101 (ECACC)

The invention now being generally described will be more readily understood by reference to the following examples, which are included merely for purposes of illustration of certain aspects and embodiments of the present invention and are not intended to limit the invention.

### EXAMPLES

As example and to illustrate the invention, CHO cells (CHO-K1 and CHO-S) were selected as appropriate cell lines to establish robust and relevant cAMP based cellular assays for the Gi-coupled receptors. Characterisation of these cell lines has confirmed that only the adenylyl cyclase isoforms VI and VII are expressed in CHO. As already mentioned both isoforms are activated by forskolin but only adenylyl cyclase isoform VI is sensitive to Gαi, adenylyl cyclase isoform VII being activated by Gas.

The adenylyl cyclase isoform VI is predominantly expressed in CHO cell lines (CHO-K1 or CHO-S) favouring the approach of using miRNA to knock-down adenylyl cyclase VII which is stimulated by forskolin but not inhibited by activated Gi protein in these cell lines.

As illustrated herein, design of miRNA for knock-down of the expression of adenylyl cyclase VII in CHO cell lines (CHO-K1 & CHO-S) has been performed. Identification of the miRNA constructs which suppress the expression of adenylyl cyclase VII in CHO cells was performed by transiently transfecting cultured CHO-K1 and CHO-S cells with simultaneously one to three different miRNA constructs and by measuring gene expression (QPCR, Western Blot) and the production of cAMP induced by Forskolin in cultured cells transfected with miRNAs. After selection of appropriate miRNA we could establish stable cell lines (CHO-K1 and CHO-S) in which the expression of adenylyl cyclase VII is suppressed by miRNA.

According to the invention, establishment of conditions in which Gi-coupled receptor specific pathways are favoured through up- or downregulation of the expression of one or more identified endogenous genes of the cell lines interfering with these specific pathways include control of the non or limited influence of the up- or downregulation of the targeted genes on the cell viability.

As illustrated herein for the CHO-K1 and CHO-S cell lines, characterisation of the relative abundance of the adenylyl cyclases VI and VII has been determined showing predominant expression of the isoform VI in CHO cell line. This favours the approach using siRNA or miRNA to knock-down adenylyl cyclase VII which is stimulated by forskolin but not inhibited by activated Gi protein.

### 1. Characterisation of Gαi proteins in CHO cell by Immunoblot

Membrane Preparation and Western Blotting. CHO-S and CHO-K1 cells were washed in PBS 37°C, harvested in a total volume of 5 ml of PBS, 0.5mM EDTA, 0.2M NaOH. Cells were pelleted 5min at 500g at 4°C and resuspended in 3mL of a buffer containing Tris/HCl 50mM, EGTA/Tris 1mM, EDTA 1mM, Trypsin 10µg/mL, Leupeptin 1µg/mL, PMSF 75µg/mL, Pepstatin 1µM. Cells were frozen at -80°C, thawed at 37°C, and ruptured on ice with by 10 passages through a 26-gauge needle. Membranes were centrifuged at 48000g during 20min at 4°C and resuspended in 20mM Hepes/NaOH, 150mM NaCl, Trypsin 10µg/mL, Leupeptin 1 µg/mL, PMSF 75µg/mL, Pepstatin 1 µM. Proteins were quantified with the Bio-Rad Protein Assay kit using bovine serum albumin (BSA) as a standard. Samples (20-40 µg of membrane protein) were resolved by SDS-polyacrylamide gel electrophoresis (4-12%), transferred to PVDF membranes, and incubated O/N at 4°C with rabbit polyclonal anti-alpha i1, mouse monoclonal anti-alpha i2 or rabbit polyclonal anti-alpha i3 antibodies (Santa Cruz) diluted 1/1000 in blocking reagent 2% (manufactuer recommended conditions) (ECL Advance kit from Amersham Biosciences, Little Chalfont, Buckinghamshire, UK). Membrane was probed with secondary anti-rabbit horseradish peroxidase conjugate (BioRad) or anti-mouse peroxydase conjugate (BioRad) diluted 1/200000 in blocking reagent 2% (manufactuer recommended conditions) (ECL Advance kit from Amersham Biosciences, Little Chalfont, Buckinghamshire, UK). Immunoreactive proteins were visualized with the ECL Advance kit from Amersham Biosciences (Little Chalfont, Buckinghamshire, UK).

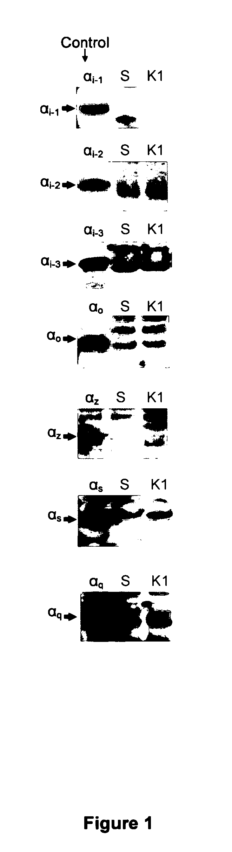

Results are shown in figure 1.

Real-time PCR. Total RNA was purified from cultured CHO-K1 and CHO-S cells using Tri-Reagent (Sigma). Using total RNA as template for reverse transcriptase, the first-strand cDNA was synthesized using RT-PCR kit (BD Bioscience Clontech) and random hexamers according to the manufacturer's instructions.

Real-time PCR was performed using Mix Sybr Green (Bio-Rad) in a iQ5 Multicolor Real-Time PCR Detection System (Bio-Rad).

The primers used for real-time PCR are as follows:
Mm_Gnai1 QT01058687 (Qiagen) for Gαi1
Mm_Gnai2 QT00140469 (Qiagen) for Gαi2
Mm_Gnai3 QT01062278 (Qiagen) for Gαi3
Mm_Gnao QT00171108 (Qiagen) for Gαo
Mm_Gapd QT00309099 (Qiagen) for GAPDH

Thermocycling conditions consisted of denaturation at 95°C for 3 min followed by 40 cycles comprising a denaturation step at 95°C for 15 s, an annealing step at 55°C for 15 s and an extension step at 72°C for 20 s. Melting curve analysis was performed with data collection from a temperature ramp from 60°C to 95°C.

### Results

GAPDH was used as an internal control gene for normalizing the PCRs.

The threshold cycle (Ct) was chosen as a point where amplification was in an exponential phase. ΔCt being the normalized Ct (Ct gene of interest - Ct control gene) value was used to determine relative abundance of gene expression.

There was no Gαi1 amplification from neither CHO-K1 nor CHO-S cells, which does correspond to the immunoblot results.

Gαi2, Gαi3 and Gαo gene expression was detected by real-time PCR in CHO-K1 and CHO-S cells. Table 1 shows the relative abundance of the expression of the Gαi protein isoforms.

**Table 1**

| Cell lines | ΔCt (Ct gene of interest - Ct control gene) | | |
|---|---|---|---|
| | Gαi3 | Gαi2 | Gαo |
| CHO-K1 | 6.71 | 14.81 | 15.72 |
| CHO-S | 7.21 | 16.2 | 17.73 |

### 2. Identification of adenylyl cyclase isoenzymes expressed in CHO cell lines by PCR amplification using degenerate primers

Total RNA was purified from culture CHO-K1 and CHO-S cell using Tri-Reagent (Sigma). Using total RNA as template for reverse transcriptase, the first-strand cDNA was synthesized using QRT-PCR SYBR Green kit (Abgen) and random hexamers according to the manufacture's instructions.

The degenerate primers (SEQ ID N° 1) were designed by matching conserved regions of the 9 families of mouse adenylyl cyclase as reported by Richard T. Premont (Methods Enzymol. 238, 116-127)
5'-CGGCAGCTCGAGAA(A/G)AT(A/C/T)AA(A/G)AC(I)AT(A/C/T)GG-3' (SEQ ID NO:1)
5'-CCGGGACTCGAGAC(A/G)TT(I)AC(I)GT(I)T(C/T)(I)CCCCA-3' (SEQ ID NO:2)

PCR amplification of the cDNA was performed with Advantage-HF 2 PCR kit (BD Science) in Thermocycle Perkin-Elmer 2400.

The PCR amplification conditions consisted of denaturation at 94°C for 1 min followed by 40 cycles comprising a denaturation step at 94°C for 15 s, an annealing step at 58°C for 30 s and an extension step at 68°C for 1 min. At the completion of the cycling reaction, an additional extension step at 68°C for 7 min was performed.

PCR products (=300 bp) were isolated by low-melting agarose gel electrophoresis and purified by GENECLEAN SPIN (Bio 101). The purified fragments were ligated into pCR2.1 vector and 4 µl of the ligation mixture were transformed into TOP 10 competent cells using a T/A cloning kit (Invitrogen).

White plasmid clones were prepared with a Minipreps DNA purification system (Eppendorf) and screened by EcoRI digestion and agarose gel electrophoresis. The positive clones, fourteen clones in CHO-K1 and twelve clones in CHO-S were sequenced.

The sequences were analysed using the BLAST search. Two different sequences were identified with high homology to published adenylyl cyclases.

Sequences SEQ ID N° 3 and SEQ ID N° 4 were aligned with known adenylyl cyclase sequences and analysed with DNASIS Software (Hitachi).

Sequence SEQ ID N° 3 is 89% (29/258 bp) homologous to that of mouse adenylyl cyclase VI (NM_007405). The homology of the deduced amino acid sequence is 99 % (1/85 aa).

Sequence SEQ ID N° 4 is 90% (27/269 bp) homologous to that of mouse adenylyl cyclase VII (NM_007406). The homology of the deduced amino acid sequence is 95% (4/80 aa).

(SEQ ID N° 3)
AGAAGATCAAAACGATAGGCAGCACCTACATGGCTGCCTCTGGGCTAAATGCCA
GCACCTATGATCAGGTGGGCCGTTCCCACATCACTGCCCTGGCAGACTATGCC
ATGAGGCTCATGGAGCAGATGAAACATATCAACGAACACTCTTTCAACAATTTCC
AGATGAAGATTGGACTGAACATGGGTCCAGTTGTGGCCGGTGTCATTGGGGCC
CGGAAGCCACAGTATGACATCTGGGGCGACACCGTCAACGTCT

(SEQ ID N° 4)
GAGAAAATAAAAACGATCGGCAGCACCTATATGGCTGCAGCAGGGCTCAGCAT
CCCCTCAGGACACGAGAACCAGGATCTGGAGCGGCAGCATGCGCACATTGGTG
TCTTGGTGGAGTTCAGCATGGCCCTGATGAGCAAGCTGGATGGCATCAACAGG
CACTCCTTCAACTCTTTCCGCCTCCGAGTTGGCATAAACCATGGGCCTGTGATT
GCTGGAGTGATTGGAGCACGAAAGCCTCAGTATGACATCTGGGGCGACACCGT
CAAC

Table 2 shows the relative abundance of adenylyl cyclase isoforms in CHO-K1 and CHO-S cells.

**Table 2**

| | CHO-K1 | CHO-S |
|---|---|---|
| Number of clone for sequencing | 14 | 12 |
| Number of clone containing adenylyl cyclase isoform VI sequence | 13 | 11 |
| Number of clone containing adenylyl cyclase isoform VII sequence | 1 | 1 |
| VII/VI % (VII %) | 1/13 (7%) | 1/12 (8%) |

### Conclusion

Two isoforms of adenylyl cyclases are expressed in CHO cell line (CHO-K1 & CHO-S) as reported in the literature (Eva V. Varga et al, 1998. Euro J Pharmacol.348, R1-R2) Predominant expression of isoform VI in CHO cell line might favour the approach using siRNA to knock-down adenylyl cyclase VII which is stimulated by forskolin but not inhibited by activated Gi protein.
Suppressing the adenylyl cyclase VII whose expression is minor in CHO cells won't influence the cell physiological function.

### 3. Amplification of adenylyl cyclase VII expressed in CHO cell lines by PCR

For siRNA (miRNA) constructions, three sets of primers were designed according to homology study between published mouse (NM_007046) and rat (XM223666) adenylyl cyclase isoform VII sequence.

For amplification of the fragment corresponding to 43-805 nt of mouse adenylyl cyclase isoform VII, primers were as follows (SEQ ID NO:5 and SEQ ID NO:6)
Sense: 5'- ATGCCAGCCAAGGGGCGCTAC-3' (SEQ ID NO:5)
Anti-sense: 5'- AGATGCTGACATTCTGGTGC-3 (SEQ ID NO:6)

For amplification of the fragment corresponding to 628-1618 nt of mouse adenylyl cyclase isoform VII, primers were as follows (SEQ ID NO:7 and SEQ ID NO:8)
Sense: 5'-CACAAGCACCAGCTGCAGGACGC-3' (SEQ ID NO:7)
Anti-sense: 5'-AGACTTGGAGCAGCAGGGCCT-3 (SEQ ID NO:8)

For amplification of the fragment corresponding to 1685-2816 nt of mouse adenylyl cyclase isoform VII, primers were as follows (SEQ ID NO:9 and SEQ ID NO:10)
Sense: 5'-TGACGAGATGCTGTCAGCCAT-3' (SEQ ID NO:9)
Anti-sense: 5'-TGCCAATGGTCTTGATCTTCTCC-3' (SEQ ID N°:10)

Total RNA was purified from culture CHO-K1 and CHO-S cell using Tri-Reagent (Sigma). Using total RNA as template for reverse transcriptase, the first-strand cDNA was synthesized using QRT-PCR SYBR Green kit (Abgen) and random hexamers according to the manufacture's instructions.

PCR amplification of the cDNA was performed with Phusion High-Fidelity DNA polymerase (Finnzymes) in Thermocycle Perkin-Elmer 2400 as follows: 98°C for 30 sec; 40 cycles of 98°C for 10 sec, 68°C for 30 sec, 72°C for 45 sec; and 72°C for 10 min.

PCR products were isolated by low-melting agarose gel electrophoresis, purified by GENECLEAN SPIN (Bio 101). The purified fragments were sequenced

### Sequencing results:

PCR fragment of CHO adenylyl cyclase isoform VII corresponding to 43-805 nt of mouse adenylyl cyclase isoform VII

(SEQ ID N° 11)
GGCCCCAACCAGGCAAAGCTCTATGAGAAGTACCGCCTCACCAGCCAGCATGG
GCCACTGTTGCTCTTGCTCCTCCTGGTGGCTGTGGCCACCTGCATCGCACTCAT
CATCATCACCTTCAGCCATGAGGATCCCCCCAGACACCAGGCTATCCTGGGCA
CTGCGTTCTTCATGCTGACGCTGTTTGTGGCTCTCTATGTGCTGGTATATGTTGA
GTGCCTGGTGCGGCGATGGCTGTGGGCCTTGGCTCTACTCACCTGGGCCTGCC
TCATGATGCTCGGCTTTGTGCTGGTGTGGGACTCCTGGAAGAATGATGCCTGTG
CGTGGGAGCAGGTGCCTTTCTTCCTGTTCATTGTCTTTGTGGTGTATGCATTACT

GCCCCTCAGCACGAGGGCAGCCATCACAGTGGGGATGGTTTCCACTGTTTCCC
ACCTCCTGATGTTTGGAGCTGTGACAGGAGCCTTCAAGATGTCCGTGTCCGGA
GCACAGCTGGCGCTGCAGCTCCTGGCCAATGCCATTATCCTCCTGGGTGGGAA
CTTCACGGGTGCCTTCCACAAGCACCAGCTGCAGGATGCTTCCCGGACCTCT
TCATCTACACAGTCAAGTGCATCCAGATCCGCCGGAAGCTGCGTGTGGAGAAG
CGCCAGCAGGAGAACCTGCTGCTTTCGGTGCTCCCAGCTCACATATCCATGGG
CATGAAGCTGGCCATCATTGAGCGCCTCAAAGAGGGTGGTGACCACCGCCACA
TGCCTGACAACAACT

PCR fragment of CHO adenylyl cyclase isoform VII corresponding to 628-1618 nt of mouse adenylyl cyclase isoform VII

(SEQ ID N° 12)
CTCTTCATCTACACAGTCAAGTGCATCCAGATCCGCCGGAAGCTGCGTGTGGAG
AAGCGCCAGCAGGAGAACCTGCTGCTTTCGGTGCTCCCAGCTCACATATCCAT
GGGCATGAAGCTGGCCATCATTGAGCGCCTCAAAGAGGGTGGTGACCACCGCC
ACATGCCTGACAACAACTTTCACAGCCTCTATGTCAAGCGGCACCAGAATGTCA
GTATCTTGTATGCAGACATTGTGGGCTTCACACGGCTGGCCAGTGACTGCTCGC
CCAAGGAGCTGGTGGTGGTACTCAACGAGCTGTTCGGGAAATTTGACCAGATT
GCCAAGGCCAATGAGTGCATGCGGATCAAGATCCTGGGTGACTGCTACTACTG
TGTGTCAGGCCTGCCTGTGTCGCTGCCCACCCATGCCCGCAACTGCGTGAAGA
TGGGTCTGGACATCTGTGAGGCCATTAAGCAGGTTCGTGAGGCCACAGGAGTG
GACATCAGCATGCGTGTGGGTATCCACTCCGGGAATGTTCTGTGTGGGGTCAT
CGGGCTCCGCAAGTGGCAGTATGATGTGTGGTCCCATGATGTGTCCCTGGCCA
ACAGGATGGAGGCAGCTGGAGTCCCTGGCCGGGTGCACATCACAGAGGCGAC
ACTGAACCACCTGGACAAGGCATACGAGGTGGAGGATGGGAACGGGCAGCAG
CGTGACCCCTATCTGAAAGAGATGAACATCCGAACCTACCTGGTGATCGACCCC
CGGAGCCAGCAGCCGCCTCCACCCAGCCATCACCTCCCCAAGCCCAAGGGGG
ATGCAACCCTGAAGATTCGGGCTTCAGTGCGTGTAACGCGCTATCTCGAGTCCT
GGGGGGCAGCACGGCCCTTCGCACACCTCAACCACCGGGAGAGTGTGAGCAG
CAGCGAGACCCCCAGTCCCACTGGACAGAGGCCAAAAGCCACTCCTCTGCGCC
GACACCGTGCCATTGACAGGAGTACATCCCCCAAGGGGCGCTTGGAGGATGAC
TGTGATGACGAGATGCTCTCGGCCAT

PCR fragment of CHO adenylyl cyclase isoform VII corresponding to 1685-2816 of mouse adenylyl cyclase isoform VII

(SEQ ID N° 13)
CCTGCTGCTCCAAGTCCGATGACTTCCACACCTTTGGTCCCATCTTCCTGGAGA
AGGGCTTTGAGCGTGAGTATCGCCTGGTGCCTATCCCCGGGCTCGCTACGAC
TTTGCCTGTGCCAGCCTTGTCTTCATCTGCATCCTGCTTGTCCACCTTCTTGTGA
TGCCCAGGATGGCCACTCTGGGCGTGTCCTTTGGACTGGTGGCCTGCCTGCTG
GGGCTGGTGCTGAGTTTCTGCTTTGCTACTGAGTTCTCAAGGTGCTTTCCAGCC
CGAGGTACACTCCAGGCCATCTCGGAGAGTGTGGAGACACAGCCCCtGCTCAG
GCTATCCTTGGTTGTGCTGACTGTTGGTAGCCTACTGACTGTTGCCATCATCAA
CATGCCACTGATGCTTAGCCCAGGCCCAGAGTGGCCAGGAGGCAATGAGACAA
GCTCGCTGGCTGCAAGGAATAGAGTTGGAATCCCGTGTGAGCTCCTCCTGTACT
ACACCTGCAGCTGCATCCTGGGCTTCATCTCGTGCTCCGTTTTCCTGCGGATGA
GCCTGGAACTGAAGGCCCTGCTGCTGACAGTGGCCTTGGTGGCTTACCTGCTG
CTCTTCAACCTCTCCCCCTGCTGGCATGTTCCAGGCAACAGCACTGAAACCAAC
AGTACACACAGGACACAAACACTCCTGCCTGCTGGACAAAGCACACCCAGCCA
CACCCTTGCTCTGGGGGCTCAGGTGACTGCCTCCTCCCCTGGCAGATGTTTAG
AGAAAGATCTGAAGACCATGATCAACTTCTACCTGGTCCTGTTCTATGCCACCCT
CATCTTGCTGTCTAGACAGATTGACTACTATTGCCGCTTGGATTGTCTGTGGAAG
AAGAAGTTCAAGAAAGAGCATGAGGAGTTTGAAACAATGGAGAATGTGAACCGC
CTCCTCCTGGAAAATGTTCTGCCAGCGCATGTGGCTGCCCACTTCATTGGCGAC
AAGGCGGCTGAGGACTGGTACCATCAGTCTTATGACTGTGTCTGTGTCATGTTT
GCCTCGGTTCCGGACTTCAAAGTGTTCTACACTGAGTGCGATGTCAACAAAGAA
GGGTTGGAGTGCCTTCGCCTGCTGAATGAGATCATTGCTGATTTtGACGAGCTG
CT

The sequences SEQ ID N° 11, SEQ ID N° 12 and SEQ ID N° 13 were aligned with known adenylyl cyclase sequences and analysed with DNASIS Software (Hitachi) and used as the templates for siRNA(miRNA) design.

### 4. miRNA design

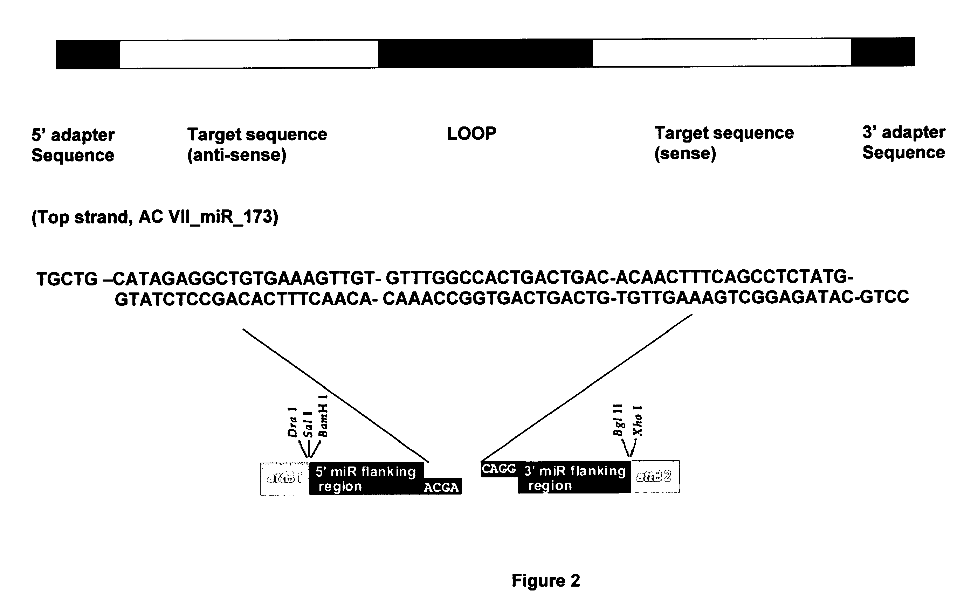

Using the Invitrogen BLOCK-iT™ RNAi Designer, eight miRNAs (SEQ ID N° 14 to 21) were synthesized and cloned into pcDNA6.2-GW/miR vector (Invitrogen). Figure 2 shows an example of the construction.

In order to demonstrate reduction of the target RNA, CHO-K1 cells were transfected with an appropriate vector.

The miRNA targeted sequences for knock-downing CHO- adenylyl cyclase isoform VII (noted ACVII_miR_NNN) are as follows:
ACVII_miR_173 : ACAACTTTCAGCCTCTATG (SEQ ID N° 14)
ACVII_miR_220: TGGTGCTGTTTCTGCTTTG (SEQ ID N° 15)
ACVII_miR_456: TGACAGGACTTCAAGATGT (SEQ ID N° 16)
ACVII_miR_553: TATGATGTGGTCCCATGAT (SEQ ID N° 17)
ACVII_miR_619: TTCCAGGCCAGCACTGAAA (SEQ ID N° 18)
ACVII_miR_1071: AAAGAAGGTGGAGTGCCTT (SEQ ID N° 19)
ACVII_miR_2102 : TCCTCCTGCTACACCTGCA (SEQ ID N° 20)
ACVII_miR_2260 : AGCACTGACCAACAGTACA (SEQ ID N° 21)

### 5. Identification of miRNA which suppress the expression of adenylyl cyclase VII in CHO cells.

### Transfection

CHO-K1 cells were plated in a 6 wells plate, 500 000 cells per well. 24 hours after plating, the cells were washed by serum-free DMEM medium. The ratio between miRNA and Lipofectamine 2000 for constructing miRNA/lipid complex was 1:2.5. The quantity of miRNA used for transfection was 1.0 µg per well for each construct and miRNA negative control prepared from Block-iT Pol II miRNAi kit (invitrogen). 6 hours after transfection, the medium was changed to DMEM with 5% dialysed serum. 48 hours later, total RNA was prepared using TriReagent (Sigma). cDNA was synthesized by RT-PCR kit (Clontech).

Using conventional polymerase chain reaction to quantify adenylyl cyclase VII messenger RNA in the cultured cells treated by miRNAs.

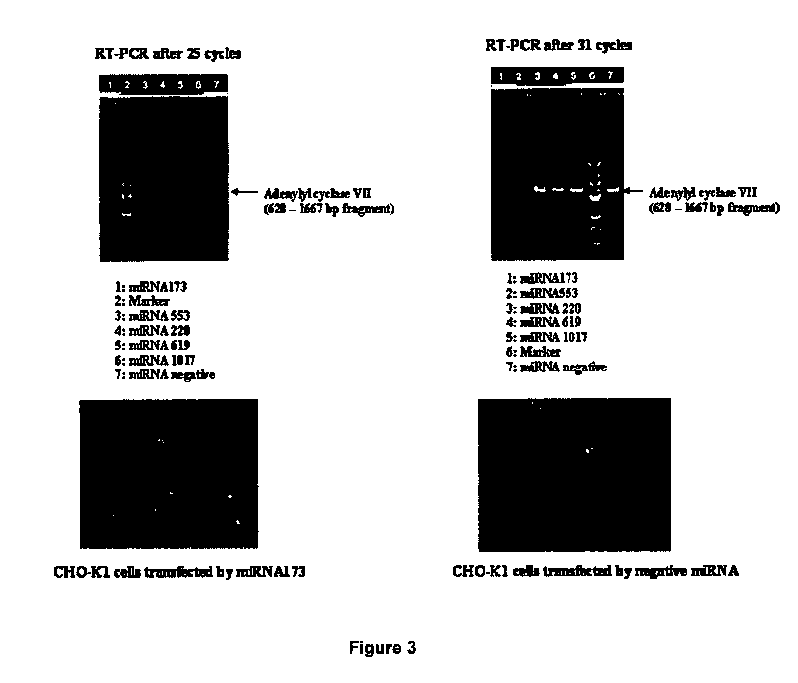

Figure 3 shows the suppressive effect of different miRNAs on the expression of adenylyl cyclase VII by Polymerase Chain Reaction (PCR) at 48 hours after transient transfection in CHO-K1 cells and the morphologies of CHO-K1 cells transfected by miRNA 173 and control miRNA.

Conclusion:
1: miRNA 173 inhibits more than 90% of expression of adenylyl cyclase VII in CHO-K1 cells after 48 hours of transient transfection.
2: No significant morphologic difference between the cells treated by miRNA 173 or negative miRNA has been observed.

### 6. cAMP assay

cAMP measurements on CHO-K1 cells that stably expressed the human muscarinic M4 or the human dopamine D4.4 receptor were performed via the HTRF (homogeneous time-resolved fluorescence) technology (CIS Bio, France). The principle of these cAMP assays is based on a competition between cAMP produced by cells and cAMP-XL665 conjugate for the binding with monoclonal anti-cAMP-cryptate conjugate. The HTRF® conjugates are prepared in lysis buffer so that the cell lysis and the detection step can be performed simultaneously.

Preparation of cells and compounds. CHO-K1 stably expressing the human muscarinic M4 or the human dopamine D4.4 receptor were cultured under standard conditions. Cells were harvested by EDTA, washed once in phosphate buffered saline and resuspended in assay buffer (HBSS containing 0.1 % glucose and 0.5 mM IBMX). The cells were dispensed into a black 96 half-well plate at a cell density of 10,000 - 15,000 cells. After incubation for 30 min at room temperature or at 37°C, an equal volume of assay buffer containing forskolin, then acetylcholine or quimpirole was added to the cells. After incubation for 30 min at room temperature or 37°C, cAMP detection was performed following the protocol described above. The plate was incubated for one more hour at room temperature and then measured on a RUBYstar^{a} (BMG Labtechnologies) time-resolved fluorescence reader.

### Results:

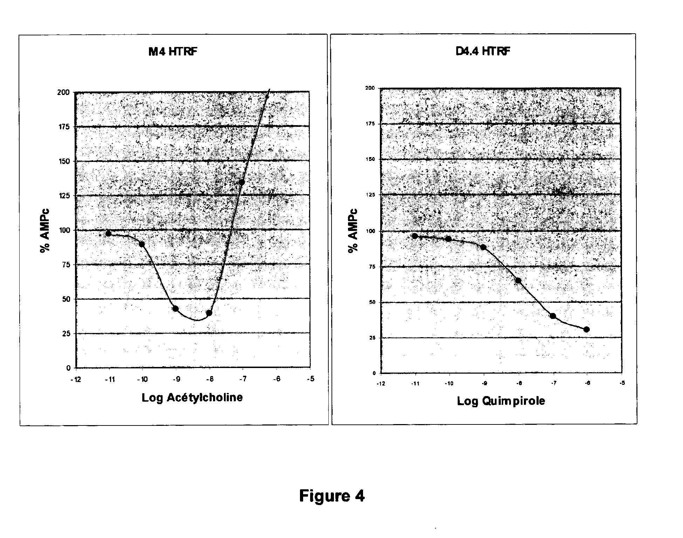

The EC50 values for acetylcholine or quimpirole were calculated from the dose response curves by Hill as shown in figure 4. CHO-K1 cells were exposed to increasing concentrations of acetylcholine or quimpirole ranging from 0,01 nM to 10 µM in the presence of 30 µM forskolin.

## Claims

1. A method for screening, identifying or selecting test compounds using an in vitro assay based on cAMP levels modulated by Gi-coupled receptors expressed in a cell comprising the steps of:
(a) optionally identifying, in a selected cell, the Gαi and adenylyl cyclase isoforms which are involved in the modulation of the intracellular cAMP levels,
(b) favouring the influence of adenylyl cyclase isoform(s) stimulated by forskolin and sensitive to inhibition by Gαi relative to adenylyl cyclase isoform(s) stimulated by forskolin but not sensitive to inhibition by Gαi in said selected cell;
(c) contacting said cell with forskolin and a test compound; and
(d) measuring cAMP levels resulting from contacting said test compound with said cell.

2. The method according to claim 1, wherein favouring the influence of adenylyl cyclase isoform(s) stimulated by forskolin and sensitive to inhibition by Gαi relative to adenylyl cyclase isoform(s) sensitive to forskolin but not sensitive to inhibition by Gαi is achieved by specifically inactivating the adenylyl cyclase isoform(s) stimulated by forskolin but not sensitive to inhibition by Gαi protein.

3. The method according to claim 2, wherein said inactivation is obtained by downregulating the expression of said adenylyl cyclase isoform(s) stimulated by forskolin but not sensitive to inhibition by Gαi protein.

4. The method according to claim 3, wherein said adenylyl cyclase isoform(s) stimulated by forskolin but not sensitive to inhibition by Gαi expressed in said selected cell are downregulated by specific RNAi.

5. The method according to claim 4, wherein said specific RNAi are specific miRNA.

6. The method according to any one of claims 1 to 5, wherein said selected cell is a eukaryotic cell selected from a mammalian cell or a yeast cell or an established cell line selected from CHO lines, HEK lines or the insect SF9 lines.

7. The method according to claim 6, wherein said selected cell is the CHO-K1 or CHO-S cell line.

8. The method according to claim 7, wherein the adenylyl cyclase VII isoform is knock-down by using specific miRNA.

9. The method according to any one of claim 1-8, wherein cAMP levels resulting from contacting said test compound with said cell is performed via technologies using read out as fluorescence or luminescence.

10. An assay system or kit for measuring the modulation of cAMP levels by test compounds that specifically interact with and modulate the activity of a cellular receptor, said assay system or kit comprising cells which mainly express adenylyl cyclase isoform(s) stimulated by forskolin and sensitive to inhibition by Gαi.

11. The assay system or kit according to claim 10, wherein said cell stably co-express i) nucleic acid sequences which code for a specific Gαi protein or a specific adenylyl cyclase isoform(s) stimulated by forskolin and sensitive to inhibition by Gα i protein (ii) and specific miRNA that target adenylyl cyclase transcripts coding for adenylyl cyclase isoform(s) stimulated by forskolin and not sensitive to inhibition by Gα i protein.

12. A recombinant cell engineered from a selected cell expressing both adenylyl cyclase isoform(s) stimulated by forskolin and sensitive to Gαi and adenylyl cyclase isoform(s) stimulated by forskolin and not sensitive to Gαi, wherein the influence of adenylyl cyclase isoform(s) stimulated by forskolin and sensitive to Gαi is favoured relative to the influence of adenylyl cyclase isoform(s) stimulated by forskolin and not sensitive to Gαi.

13. The recombinant cell according to claim 12, wherein expression of adenylyl cyclase isoform(s) stimulated by forskolin and sensitive to Gαi is upregulated.

14. The recombinant cell according to claim 12, wherein adenylyl cyclase isoform(s) stimulated by forskolin and not sensitive to Gαi are inactivated.

15. The recombinant cell according to claim 14, wherein the gene(s) coding for adenylyl cyclase isoform(s) stimulated by forskolin and not sensitive to Gαi are deleted.

16. The recombinant cell according to claim 14, wherein the expression of adenylyl cyclase isoform(s) stimulated by forskolin and not sensitive to Gαi is downregulated by RNAi.

17. The recombinant cell according to claim 16, wherein said cell is stably transformed with a recombinant DNA vector comprising (i) engineered pre-miRNA primary transcripts, and ii) nucleic acid sequences which code for a specific Gαi protein or a specific adenylyl cyclase isoform(s) stimulated by forskolin and sensitive to inhibition by Gαi protein, stably co-expressed in said recombinant cell.

18. The recombinant cell according to claim 17, which is engineered to co-express a specific adenylyl cyclase isoform(s) stimulated by forskolin and sensitive to inhibition by Gαi protein and specific miRNA that target adenylyl cyclase transcripts coding for adenylyl cyclase isoform(s) stimulated by forskolin and not sensitive to inhibition by Gαi protein.

19. The recombinant cell according to any one of claims 12 to 18, which is a mammalian cell.

20. The recombinant cell according to claim 19, which is a CHO cell line.

21. A use of a recombinant cell as defined in any one claims 12 to 20, as a tool for screening, identifying or selecting potential ligands of Gi-coupled receptors.

22. The assay system or kit as defined in claim 10 or 11, wherein said cells are as defined in anyone of claims 12-20.
